(19) Europäisches Patentamt
European Patent Office
Office européen des brevets

(11) Veröffentlichungsnummer: **0 213 601**
**A2**

(12) # EUROPÄISCHE PATENTANMELDUNG

(21) Anmeldenummer: 86111842.0

(22) Anmeldetag: 27.08.86

(51) Int.Cl.[4]: A 01 N 47/44
//(A01N47/44, 33:12)

(30) Priorität: 03.09.85 DE 3531356

(43) Veröffentlichungstag der Anmeldung:
11.03.87 Patentblatt 87/11

(84) Benannte Vertragsstaaten:
DE GB NL

(71) Anmelder: HOECHST AKTIENGESELLSCHAFT
Postfach 80 03 20
D-6230 Frankfurt am Main 80(DE)

(72) Erfinder: Wallhäusser, Karl Heinz, Prof. Dr.
Lessingstrasse 20
D-6238 Hofheim am Taunus(DE)

(72) Erfinder: Hille, Martin, Dr.
In den Eichen 46
D-6237 Liederbach(DE)

(72) Erfinder: Bücking, Hans-Walter, Dr.
In den Padenwiesen 30
D-6233 Kelkheim (Taunus)(DE)

(72) Erfinder: Hofinger, Manfred, Dr.
Hoher Göll Weg 7
D-8269 Burgkirchen(DE)

(54) **Mikrobiozide Mittel auf Basis von Alkyl-di-guanidiniumsalzen.**

(57) Die mikrobioziden Mittel bestehen im wesentlichen aus 5 bis 50 Gew.-% des Salzes eines Alkyl-di-guanidins der Formel

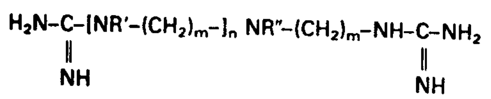

worin R' und R" Wasserstoff oder $C_8$-$C_{18}$-Alkyl bedeuten, wobei R' und R" nicht gleichzeitig Wasserstoff sein dürfen, m eine Zahl von 2 bis 6 und n 0 oder 1 bedeuten,
5 bis 50 Gew.-% einer quartären Ammoniumverbindung der Formel

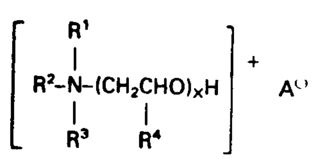

wobei $R^1$ $C_8$-$C_{22}$-Alkyl oder $C_8$-$C_{22}$-Alkenyl, $R^2$ und $R^3$ $C_1$- $C_4$-Alkyl oder $C_2$-$C_3$-Hydroxyalkyl und $R^4$ Wasserstoff oder Methyl oder $R^1$ und $R^2$ $C_8$-$C_{12}$-Alkyl, $R^3$ $C_1$-$C_4$-Alkyl oder $C_2$-$C_3$-Hydroxyalkyl, $R^4$ Wasserstoff oder Methyl, X in beiden Fällen eine Zahl von 1 bis 3, vorzugsweise von 1 bis 2, insbesondere 1,5 und $A^{\ominus}$ ein Anion einer Carbonsäure aus der Gruppe Propionsäure, Milchsäure, Glykolsäure, Tartronsäure, Aepfelsäure, Weinsäure, Zitronensäure, Maleinsäure und Benzoesäure bedeuten und der Rest ist Wasser und/oder ein niederer Alkohol oder ein wasserlösliches Glykol.

# Mikrobiozide Mittel auf Basis von Alkyl-di-guanidiniumsalzen

Es ist bereits bekannt, daß Alkyl-di-guanidiniumsalze eine gute bakterizide und fungizide Wirkung besitzen (DE-C 1249457). Wegen der teilweise unbefriedigenden Löslichkeit in Wasser und der schlechten Verträglichkeit, besonders mit hartem oder kochsalzhaltigem Wasser, ist ihre Einsatzmöglichkeit aber beschränkt.

Um die Löslichkeit und Stabilität in kochsalzhaltigem oder hartem Wasser zu verbessern, ist es bereits bekannt, sie mit quartären Ammoniumverbindungen bzw. Phosphoniumverbindungen oder mit Fettalkyldiaminsalzen zu kombinieren. Es ist jedoch hier von Nachteil, daß die mikrobiozide Wirkung der Alkyl-di-guanidiniumsalze durch diese Emulgatoren negativ beeinfluß wird. Weiterhin ist bekannt (EP-A 3999), daß Kombinationen von Alkyl-di-guanidiniumsalzen und Polyoxyethylen-/Polyoxypropylen-Blockpolymerisaten stabile Formulierungen mit verbesserter mikrobiozider Wirkung ergeben. Solche Formulierungen weisen aber einen unzureichenden Stockpunkt auf.

Gegenstand der Erfindung sind neue, verbesserte mikrobiozide Mittel bestehend im wesentlichen aus 5 bis 50, vorzugsweise 20 bis 30 Gew.-% des Salzes eines Alkyl-di-guanidins der Formel

$$H_2N-\underset{\underset{NH}{\|}}{C}-[NR'-(CH_2)_m-]_nNR''-(CH_2)_m-NH-\underset{\underset{NH}{\|}}{C}-NH_2$$

worin R' und R" Wasserstoff oder $C_8$-$C_{18}$-Alkyl bedeuten, wobei R' und R" nicht gleichzeitig Wasserstoff sein dürfen, m eine Zahl von 2 bis 6 und n 0 oder 1 bedeuten,
5 bis 50, vorzugsweise 20 bis 30 Gew.-% einer quartären Ammoniumverbindung der Formel

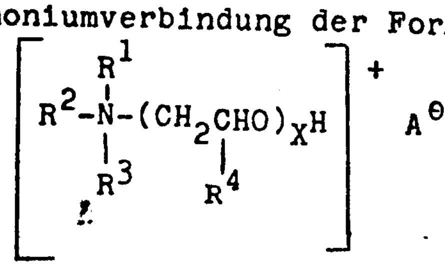

wobei $R^1$ $C_8$-$C_{22}$-Alkyl oder $C_8$-$C_{22}$-Alkenyl, $R^2$ und $R^3$ $C_1$-$C_4$-Alkyl oder $C_2$-$C_3$-Hydroxyalkyl und $R^4$ Wasserstoff oder Methyl oder $R^1$ und $R^2$ $C_8$-$C_{12}$-Alkyl, $R^3$ $C_1$-$C_4$-Alkyl oder $C_2$-$C_3$-Hydroxyalkyl, $R^4$ Wasserstoff oder Methyl, X in beiden Fällen eine Zahl von 1 bis 3, vorzugsweise von 1 bis 2, insbesondere 1,5 und $A^\ominus$ ein Anion einer Carbonsäure aus der Gruppe Propionsäure, Milchsäure, Glykolsäure, Tartronsäure, Aepfelsäure, Weinsäure, Zitronensäure, Maleinsäure und Benzoesäure bedeuten und der Rest ist Wasser und/oder ein niederer Alkohol oder ein wasserlösliches Glykol.

Bevorzugt sind solche quartären Ammoniumverbindungen, worin $R^1$ $C_{12}$-$C_{18}$-Alkyl oder $C_{12}$-$C_{18}$-Alkenyl, $R^2$ und $R^3$ Methyl, $R^4$ Wasserstoff oder $R^1$ und $R^2$ $C_8$-$C_{12}$-Alkyl, $R^3$ Methyl, $R^4$ Wasserstoff und $A^\ominus$ das Anion der Propionsäure Benzoesäure oder Milchsäure bedeuten.

Die verwendeten Alkyl-di-guanidine lassen sich nach schon bekannten Verfahren, z.B. durch Reaktion von Diaminen der Formel

$$H[R'N-(CH_2)_m-]_nNR''-(CH_2)_m-NH_2$$

in der R', R", m und n die oben angegebene Bedeutung besitzen, mit Cyanamid oder S-Alkylisothioharnstoff herstellen.

Zur Salzbildung kommen sowohl ein- oder mehrwertige anorganische oder organische Säuren in Betracht, beispielsweise Schwefelsäure, Salpetersäure, Phosphorsäure, Ameisensäure oder Salzsäure. Außerdem können zur Salzbildung auch organische Säuren wie z.B. Essigsäure, Propionsäure, Milchsäure und auch höher molekulare aliphatische Carbonsäuren wie z.B. Laurinsäure, Stearinsäure, Ölsäure u.ä. oder deren Gemische eingesetzt werden. Anstelle einheitlicher Individuen können auch Mischungen der genannten Verbindungen, gegebenenfalls auch zusammen mit anderen Mikrobioziden verwendet werden. Die oben beschriebenen quartären Ammoniumverbindungen lassen sich nach an sich bekannten Verfahren herstellen, wie z.B. in der DE-A 3 319 509 beschrieben.

Die erfindungsgemäßen mikrobioziden Mittel, die durch einfaches Vermischen der angegebenen Komponenten hergestellt werden, zeichnen sich durch eine gute Wasserlöslichkeit aus, und sind gut vertraglich mit hartem oder kochsalzhaltigem Wasser. Besonders hervorzuheben ist die verbesserte mikrobiozide Wirkung im Vergleich zu den bisher bekannten Formulierungen mit quartären Ammoniumverbindungen. Weiterhin ist zu erwähnen, daß die erfindungsgemäßen Mischungen einen niedrigen Stockpunkt aufweisen (unterhalb -40°C). Dadurch können sie besonders in kalten Regionen eingesetzt werden. Außerdem zeigen diese Formulierungen gegenüber herkömmlichen Quats einen verbesserten Korrosionseffekt. Handelsübliche quartäre Ammoniumchloride zeigen nämlich stark korrosive Eigenschaften gegen die üblichen Metalle, wie z.B. Eisen, Stahl, V2A, V4A.

Folgende Beispiele dienen zur Erläuterung der Erfindung:

Beispiel 1

25 Teile Cocospropylendiamin-di-guanidiniumdiacetat
25 Teile Didecyl-methyl-oxiethyl-ammoniumpropionat
30 Teile Isobutanol
15 Teile Polyethylenglykol
5 Teile Wasser

Diese Formulierung ist unter den Bedingungen des Schaukeltests stabil. Sie läßt sich mit Wasser in jedem Verhältnis klar löslich weiterverdünnen. Der Stockpunkt liegt unter -50°C. Die mikrobioziden Effekte kann man der Tabelle entnehmen.

Beispiel 2

25 Teile Cocospropylendiamin-di-guanidinumacetat
25 Teile Didecyl-methyl-oxiethyl-ammoniumpropionat

0213601

20 Teile Isobutanol
20 Teile Glykol
5 Teile Isopropanol

Ergebnisse sind vergleichbar mit Beispiel 1.

Beispiel 3

25 Teile Cocospropylendiamin-di-guanidiniumdiacetat
25 Teile Didecyl-methyl-oxiethyl-ammoniumpropionat
25 Teile Glykol
25 Teile Isobutanol

Ergebnisse sind vergleichbar mit Beispiel 1.

Beispiel 4

25 Teile Bisguanidiniumderivat der Formel

$$H_2N-\underset{\overset{\|}{NH}}{C}-NH-(CH_2)_3-\underset{\overset{|}{R}}{N}-(CH_2)_3-NH-\underset{\overset{\|}{NH}}{C}-NH_2$$

R = Cocosalkyl
25 Teile Didecyl-methyl-oxiethyl-ammoniumpropionat
25 Teile Isopropanol
25 Teile Glykol

Ergebnisse sind vergleichbar mit Beispiel 1.

Beispiel 5

25 Teile Cocospropylendiamin-di-guanidiniumdiacetat
25 Teile Didecyl-methyl-oxiethyl-ammoniumpropionat
50 Teile Polyglykol 400.

Beispiel 6

25 Teile Cocospropylendiamin-di-guanidiniumacetat
25 Teile Soja-alkyl-dimethyl-oxyethyl-ammoniumpropionat
37 Teile Isobutanol
100 Teile Glykole/Wasser

Vergleichsprodukt (handelsüblich)

25 Teile Cocospropylendiamin-di-guanidiniumdiacetat
25 Teile Soja-trimethyl-ammoniumchlorid
25 Teile Isopropanol
25 Teile Wasser

Ergebnis:

Bei dem Vergleichsprodukt ist die Wasserverdünnbarkeit gut, im Vergleich zu den erfindungsgemäßen Beispielen 1 bis 6 sind die mikrobioziden Effekte reduziert. Der Kältetrübungspunkt (Stockpunkt) ist unzureichend.

Die mikrobiozide Wirkung wurde nach dem quantitativen Suspensionsversuch geprüft. Eine detaillierte Beschreibung der Versuchsdurchführung kann man der Literatur entnehmen (Zbl. Bakt. Hyg. I Abt. Orig. B 165, 355-380, 1977).

Quantitativer Suspensionsversuch

| Keimart | Beispiel 1 | | | Vergleichsprodukt | | |
|---|---|---|---|---|---|---|
| | 1h | 6h (ppm) | 24h | 1h | 6h | 24h |
| Staph. aureus | 125 | 62,5 | 15,6 | 250 | 125 | 62,5 |
| E. coli | 250 | 62,5 | 31,2 | 1000 | 125 | 62,5 |
| Pseudomonas aeruginosa | 500 | 250 | 62,5 | 1000 | 500 | 125 |
| Candida albicans | 62,5 | 15,6 | 7,7 | 250 | 31,2 | 15,6 |
| Aspergillus niger | 500 | 250 | 62,5 | 1000 | 1000 | 250 |
| Stockpunkt DIN 51583 | ca. -50°C | | | ca. -20°C | | |

Mikrobiozide Wirkung in µg/ml

| Desulfovibrio-desulfuricans | Beispiel 6 | Vergleichsprodukt |
|---|---|---|
| D1 | 6,25 | 6,25 |
| D2 | >200 | >200 |
| D3 | <3,1 | <3,1 |
| D39 | 50 | 100 |

Quantitativer Suspensionsversuch

| Keimart | Beispiel | | | Vergleichsprodukt | | |
|---|---|---|---|---|---|---|
| | 1h | 6h | 24h | 1h | 6h | 24h |
| | | (ppm) | | | (ppm) | |
| Staph. aureus | 125 | 62,5 | 15,6 | 250 | 125 | 62,5 |
| E. coli | 500 | 250 | 125 | 1000 | 125 | 62,8 |
| Pseudomonas aeruginosa | 500 | 250 | 62,5 | 1000 | 500 | 125 |
| Candida albicans | 125 | 62,5 | 31,2 | 250 | 31,2 | 15,6 |
| Aspergillus niger | 500 | 250 | 62,5 | 1000 | 1000 | 250 |
| Stockpunkt DIN 53583 | ca. -40°C | | | ca. -20°C | | |

Aus den Tabellen sind die verbesserten mikrobioziden Effekte der erfindungsgemäßen Mischungen im Vergleich zu einem handelsüblichen Produkt ersichtlich.

Um eine Keimabtötung nach dem quantitativen Suspensionsversuch zu erreichen, benötigt man im Vergleich zu dem handelsüblichen Produkt bei den erfindungsgemäßen Mischungen wesentlich geringere Einsatzkonzentrationen (ppm). Bemerkenswert ist auch der vergleichsweise niedrige Stockpunkt der erfindungsgemäßen Mischungen im Vergleich zum Handelsprodukt. Dies erlaubt einen Einsatz der erfindungsgemäßen Kombination in sehr kalten Regionen, wie sie z.B. in Sibirien auftreten, wo solche keimabtötenden Mittel auf dem Erdölsektor eingesetzt werden.

Patentansprüche

1. Mikrobiozide Mittel bestehend im wesentlichen aus 5 bis 50 Gew.-% des Salzes eines Alkyl-di-guanidins der Formel

$$H_2N-C(=NH)-[NR_1-(CH_2)_m-]_nNR''-(CH_2)_m-NH-C(=NH)-NH_2$$

worin R' und R" Wasserstoff oder $C_8$-$C_{18}$-Alkyl bedeuten, wobei R' und R" nicht gleichzeitig Wasserstoff sein dürfen, m eine Zahl von 2 bis 6 und n 0 oder 1 bedeuten, 5 bis 50 Gew.-% einer quartären Ammoniumverbindung der Formel

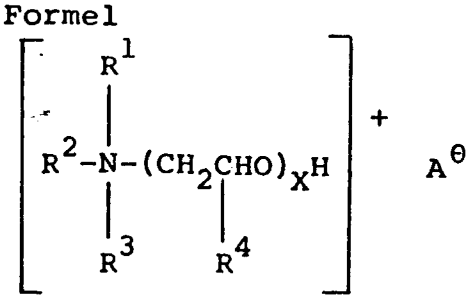

wobei $R^1$ $C_8$-$C_{22}$-Alkyl oder $C_8$-$C_{22}$-Alkenyl, $R^2$ und $R^3$ $C_1$-$C_4$-Alkyl oder $C_2$-$C_3$-Hydroxyalkyl und $R^4$ Wasserstoff oder Methyl oder $R^1$ und $R^2$ $C_8$-$C_{12}$-Alkyl, $R^3$ $C_1$-$C_4$-Alkyl oder $C_2$-$C_3$-Hydroxyalkyl, $R^4$ Wasserstoff oder Methyl, X in beiden Fällen eine Zahl von 1 bis 3, vorzugsweise von 1 bis 2, insbesondere 1,5 und $A^\ominus$ ein Anion einer Carbonsäure aus der Gruppe Propionsäure, Milchsäure, Glykolsäure, Tartronsäure, Aepfelsäure, Weinsäure, Zitronensäure, Maleinsäure und Benzoesäure bedeuten und der Rest ist Wasser und/oder ein niederer Alkohol oder ein wasserlösliches Glykol.

2. Mikrobiozide Mittel nach Anspruch 1, dadurch gekennzeichnet, daß sie eine quartäre Ammoniumverbindung enthalten, worin $R^1$ $C_{12}$-$C_{18}$-Alkyl oder $C_{12}$-$C_{18}$-Alkenyl, $R^2$ und $R^3$ Methyl, $R^4$ Wasserstoff oder $R^1$ und $R^2$ $C_8$-$C_{12}$-Alkyl, $R^3$ Methyl, $R^4$ Wasserstoff und $A^\ominus$ das Anion der Propionsäure, Benzoesäure oder Milchsäure bedeuten.